(19) Europäisches Patentamt

European Patent Office

Office européen des brevets

(11) Veröffentlichungsnummer: **0 351 545 B1**

(12) **EUROPÄISCHE PATENTSCHRIFT**

(45) Veröffentlichungstag der Patentschrift:
18.12.91 Patentblatt 91/51

(51) Int. Cl.⁵: **A61F 2/32**

(21) Anmeldenummer: 89110735.1

(22) Anmeldetag: 14.06.89

(54) Gelenkprothese.

(30) Priorität: 16.07.88 DE 3824243

(43) Veröffentlichungstag der Anmeldung:
24.01.90 Patentblatt 90/04

(45) Bekanntmachung des Hinweises auf die
Patenterteilung:
18.12.91 Patentblatt 91/51

(84) Benannte Vertragsstaaten:
AT BE DE ES FR GB IT LU NL

(56) Entgegenhaltungen:
WO-A-86/01394
DE-A- 3 200 340
DE-B- 2 334 643
FR-A- 1 416 534
US-A- 3 818 514

(73) Patentinhaber: Friedrichsfeld
Aktiengesellschaft Keramik- und
Kunststoffwerke
Steinzeugstrasse 50
W-6800 Mannheim 71 (DE)

(72) Erfinder: Meiss, Ludwig, Prof.Dr.
Reichskanzlerstrasse 23 a
W-2000 Hamburg (DE)
Erfinder: Hund, Walter
Ringstrasse 20a
W-7602 Oberkirch (DE)
Erfinder: Vizethum, Freimut, Dr.
Holunderweg 2
W-6830 Schwetzingen (DE)

(74) Vertreter: Klose, Hans, Dipl.-Phys. et al
Kurfürstenstrasse 32
W-6700 Ludwigshafen (DE)

## Beschreibung

Die Erfindung bezieht sich auf eine Gelenkprothese, insbesondere Hüftgelenkprothese, gemäß den im Oberbegriff des Patentanspruchs 1 angegebenen Merkmalen.

Aus der DE-B-2411617 ist eine derartige Gelenkprothese bekannt, welche als eine zementfrei implantierbare Prothese zum Ersatz der Hüftgelenkpfanne ausgebildet ist. Diese Gelenkprothese enthält eine Keramik-Kalotte mit einer inneren, halbkugelartig ausgebildeten Gelenkfläche. Diese Kalotte ist außen von einer Kunststoffummantelung umgeben, welche an den Rändern Wülste aufweist, um die Keramik-Kalotte einschnappbar an der Kunststoffummantelung zu fixieren. Die Kunststoffummantelung ihrerseits ist gleichfalls mit einem Einschnappmechanismus in einem äußeren Pfannenteil fixiert, welcher mittels eines Gewindeprofils im Knochen verankert werden kann. Mit der Kunststoffummantelung soll eine Stoßdämpfung als Ausgleich zu den sehr harten Keramik-Gelenkoberflächen bewirkt werden. Die Kunststoffummantelung ist nicht der radial innenliegenden Oberfläche der Pfanne zugeordnet und dient ferner zur Fixierung und Stoßdämpfung für die gesamte Lebensdauer der Gelenkprothese.

Aus der DE-B-2747867 ist eine Gelenkprothese mit einer zweiteiligen Gelenkpfanne bekannt. Eine innere Gelenkpfanne ist drehfest oder drehbar in eine äußere Gelenkpfanne eingesetzt. Der als Kugelkörper ausgebildete Gelenkkopf wird von der inneren Gelenkpfanne aufgenommen, welche aus einem Kunststoff besteht und in einem Eingangsabschnitt eine ringförmige Nut sowie eine elastisch verformbare ringförmige Lippe aufweist. Diese ringförmige Lippe wirkt wie eine Sperre, die einer Kraft in Richtung des Schaftes bzw. der Kugelachse einen erheblichen Widerstand entgegensetzen soll, wobei andererseits zum Einsetzen nur eine vergleichsweise geringe Kraft erforderlich sein soll.

Schließlich ist aus der DE-B-3200340 eine Pfanne für eine Hüftgelenkendoprothese mit einer ringförmigen Scheibe an einem unteren Pfannenrand bekannt. Diese Scheibe ist als ein Haltering ausgebildet und ist mittels eines Klemmringes mit dem Pfannenkörper fest verbunden. Mit einem zusätzlichen Klemmring soll einem Fließen des aus Kunststoff bestehenden Halterings bei Druckbelastung entgegengewirkt werden.

Gelenkprothesen der gattungsgemäßen Art werden heute überwiegend aus Oxidkeramik, und zwar insbesondere aus hochreinem Aluminiumoxid ($AL_2 O_3$) gefertigt. Derartige Oxidkeramik-Werkstoffe oder auch vergleichbare Werkstoffe weisen eine hohe Härte auf und gewährleisten grundsätzlich eine hohe Funktionssicherheit und Lebensdauer. Im praktischen Einsatz zeigt es sich jedoch gelegentlich, daß Beschädigungen von Pfannen- oder Kugelkörper auftreten, die aufgrund der großen Härte des Werkstoffes nicht von Fremdkörpern herrühren können. Vielmehr ist davon auszugehen, daß bei der Operation, insbesondere beim Reponieren, eine unzulässig hohe Stoßbelastung zwischen Pfanne und Kugel auftritt. Besonders gefährdet ist hierbei der Pfannenrand, aus welchem beim Anschlagen der Kugel Splitter herausgeschlagen werden können, die später zu einer Beschädigung oder gar Zerstörung von Kugel oder Pfanne führen. Die hieraus resultierenden Nachteile für den Patienten liegen auf der Hand, wobei auch ein totaler Austausch mit sämtlichen operativen Risiken der Gelenkprothese bisher in Kauf genommen werden mußte.

Der Erfindung liegt daher die Aufgabe zugrunde, die Gelenkendoprothese derart weiterzubilden, daß eine Beschädigung von Kugel und/oder Pfanne zuverlässig vermieden wird und eine hohe Lebensdauer sichergestellt werden kann. Es soll ein sicherer Schutz vor und während der Operation, insbesondere beim Reponieren von Kugel und Pfanne, zuverlässig gewährleistet werden.

Die Lösung dieser Aufgabe erfolgt gemäß den im Kennzeichen des Patentanspruchs 1 angegebenen Merkmalen.

Die vorgeschlagene Gelenkendoprothese zeichnet sich durch eine einfache Konstruktion aus und ermöglicht einen sicheren Schutz von Kugel sowie Pfanne vor und während der Operation, und zwar insbesondere beim Reponieren von Kugel und Pfanne. Mittels der genannten Schutzschicht werden Stoßbeanspruchungen und ein Abplatzen von selbst kleinsten Partikeln des vergleichsweise spröden und gleichwohl harten Werkstoffes von Pfanne oder Kugel verhindert. Zweckmäßig ist die Schutzschicht als eine Scheibe ausgebildet, welche auf der die Pfanne umgebenden, bevorzugt ringförmigen Oberfläche des Pfannenkörpers angeordnet ist. Die Schutzschicht besteht hierbei zweckmäßig aus einem Kunststoff und ist mit einem Schlitz versehen, um nach dem Einschnappen der Kugel in die Pfanne abgezogen werden zu können. Der Innendurchmesser der Scheibe ist zweckmäßigerweise etwas kleiner als der Randdurchmesser der Pfanne, um ein Anschlagen der Kugel sicher zu unterbinden. Andererseits wird aufgrund der Nachgiebigkeit des im Vergleich weichen Kunststoffes der Scheibe das Eintreten der Kugel in die Pfanne ermöglicht. Die Scheibe weist darüberhinaus Ansätze auf, die formschlüssig in Löcher oder Ausnehmungen des Pfannenkörpers eingreifen und eine sichere Fixierung der Scheibe auf dem Pfannenkörper solange ermöglichen, bis nach dem Reponieren vom Chirurg die Scheibe aus der Gelenkprothese entfernt wird. Alternativ kann im Rahmen der Erfindung die Schutzschicht aus einem Material bestehen, das nach dem Einsetzen resorbiert bzw. durch die Körperflüssigkeit aufgelöst werden kann. Besonders sei hier Gelatine genannt, doch können auch andere Werkstoffe vorgesehen werden, welche vom Organismus abgebaut werden können. Derartige Materialien sind insbesondere Polylactat und Polyglycolat und andere Materialien mit vergleichbaren Eigenschaften.

Die Erfindung wird nachfolgend anhand der Zeichnung näher erläutert. Es zeigen :

Fig. 1 eine Aufsicht auf eine als Scheibe ausgebildete Schutzschicht,

Fig. 2 einen Schnitt entlang Schnittlinie II gemäß Fig. 1 zusammen mit dem Pfannenkörper,

Fig. 3 einen Schnitt durch den mit einer Schutzschicht versehenen Kugelkörper.

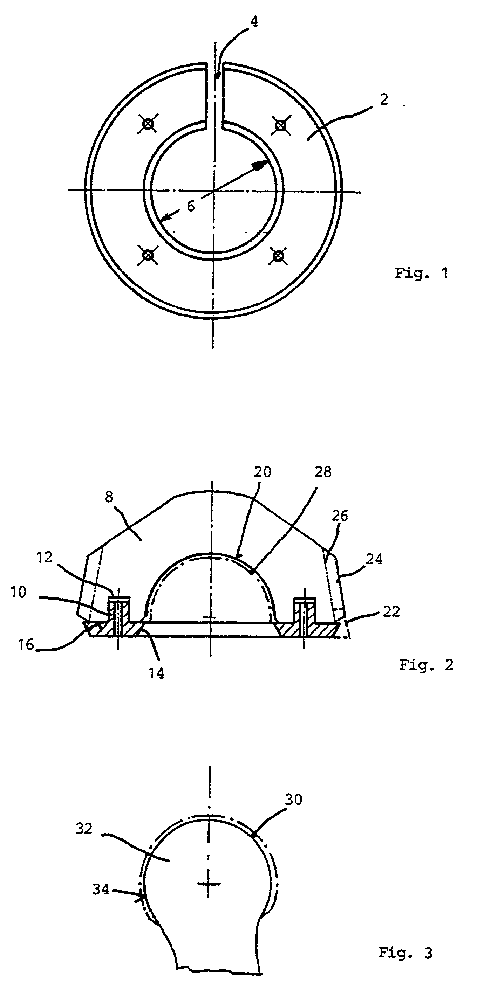

Fig. 1 zeigt in einer Aufsicht eine Scheibe 2, welche einen radialen Schlitz 4 aufweist. Die Scheibe 2 besteht aus einem im Vergleich zum Material der Gelenkprothese weichen, nachgiebigen Werkstoff und schützt den Pfannenkörper vor und während der Operation vor Beschädigungen. Der Innendurchmesser 6 wird in zweckmäßiger Weise derart vorgegeben, daß die einzusetzende Kugel nicht an den Pfannenrand anschlagen kann. Es wird damit ein zuverlässiger Schutz sowohl des Pfannenrandes als auch der Kugel gewährleistet.

Fig. 2 zeigt die Scheibe 2 zusammen mit dem Pfannenkörper 8, der aus einem im Vergleich zu der Scheibe 2 harten Werkstoff besteht. Wie ersichtlich, weist die Scheibe 2 Ansätze 10 auf, welche in entsprechende Ausnehmungen 12 des Pfannenkörpers 8 formschlüssig eingreifen. Aufgrund der nach innen zur Pfanne gerichteten konischen Ausbildung des Randes 14 der Scheibe 2 wird das Zentrieren und Einführen der Kugel erleichtert. Die Scheibe 2 liegt fest auf der Oberfläche 16 des Pfannenkörpers 8 auf. Aufgrund des formschlüssigen Ineinandergreifens der Ansätze 10 mit den korrespondierenden Ausnehmungen 12 des Pfannenkörpers wird eine funktionssichere Fixierung erreicht. Ist der Kugelkörper in die Pfanne 20 eingesetzt, so kann der Chirurg problemlos die Scheibe 2 von der Oberfläche 16 entfernen, und aufgrund des obengenannten Schlitzes über den Kugelkörper abziehen. Wichtig ist insoweit, daß die Scheibe 2 aus einem elastisch nachgiebigen Werkstoff besteht, der ein Aufweiten und ein Abziehen über den Kugelkörper hinweg ermöglicht.

Anstelle der in Fig. 2 etwa in der Mitte der radialen Breite der Scheibe 2 angeordneten Ansätze können alternativ vergleichbare Ansätze 22 radial außen angeordnet sein, wie es in Fig. 2 strichpunktiert angedeutet ist. Der Pfannenkörper 8 weist bekanntlich an seiner Außenfläche selbsteinschneidende Gewindegänge 24 zur Befestigung im Knochen auf. Zwischen den Gewindegängen 24 sind in Umfangsrichtung betrachtet Längsnuten vorhanden, welche durch die strichpunktierten Linien angedeutet sind und in welche die Ansätze 22 eingreifen, um eine formschlüssige Verbindung der Scheibe 2 mit dem Pfannenkörper 8 ermöglichen.

Ferner können zusätzlich oder alternativ die Pfanne 20 ebenso wie der Pfannenrand 14 und bedarfsweise auch die Oberfläche 16 mit einer Schicht 28 aus einem Werkstoff versehen sein, der nach der Operation von der Körperflüssigkeit abgebaut werden kann, genannt sei hier vor allem Gelatine. Die Schicht 28 ist hier stark vergrößert dargestellt.

In Fig. 3 ist der Kugelkörper 32 dargestellt, der auf seiner kugelförmigen Oberfläche 34 wiederum eine Schicht 30 aus dem obengenannten Werkstoff aufweist. Zweckmäßig sind beide Körper, also Kugelkörper 32 ebenso wie der Pfannenkörper mit der genannten Schicht versehen.

## Bezugszeichen

| | | |
|---|---|---|
| 2 | | Scheibe |
| 4 | | Schlitz |
| 6 | | Innendurchmesser |
| 8 | | Pfannenkörper |
| 10 | | Ansatz |
| 12 | | Ausnehmung |
| 14 | | Rand von 2 |
| 16 | | Oberfläche von 8 |
| 20 | | Pfanne |
| 22 | | Ansatz |
| 24 | | Gewindegang |
| 26 | | Linie |
| 28, | 30 | Schicht |
| 32 | | Kugelkörper |
| 34 | | Oberfläche von 32 |

## Patentansprüche

1. Gelenkprothese, insbesondere Hüftgelenkprothese, mit einem Kugelkörper (32) und mit einem einen ringförmigen Pfannenrand aufweisenden Pfannenkörper (8), wobei die Oberfläche (34) des Kugelkörpers (32) an einer inneren Oberfläche (20) des Pfannenkörpers (8) anliegt, dadurch gekennzeichnet, daß auf der Oberfläche (16) des Pfannenrandes eine nach dem Reponieren des Kugelkörpers (32) in den Pfannenkörper (8) zu entfernende Scheibe(2) angeordnet ist oder daß mindestens die Oberfläche (16) des Plannenvandes oder die Oberfläche (34) des Kugelkörpers (32) mit einer Schutzschicht (28, 30) versehen ist, welche aus einem vom Körper resorbierbaren Werkstoff besteht, und daß der Werkstoff der Scheibe (2) oder der Schutzschicht (28, 30) eine geringere Härte aufweist als der Werkstoff des Pfannenkörpers (8) bzw. des Kugelkörpers (32).

2. Gelenkprothese nach Anspruch 1, dadurch gekennzeichnet, daß die ringförmig ausgebildete Scheibe (2) und/ oder die Schutzschicht (28, 30) eine im wesentlichen gleichbleibende Schichtdicke aufweisen.

3. Gelenkprothese nach Anspruch 2, dadurch gekennzeichnet, daß die Scheibe (2) wenigstens einen Ansatz (10) aufweist, welcher in eine Ausnehmung (12) des Pfannenkörpers (8) formschlüssig eingreift.

4. Gelenkprothese nach Anspruch 2 oder 3, dadurch gekennzeichnet, daß die Scheibe (2) einen durchgehenden, radialen Schlitz (4) aufweist.

5. Gelenkprothese nach einem der Ansprüche 2 bis 4, dadurch gekennzeichnet, daß der Innenrand (14) der Scheibe (2) insbesondere nach Art eines Konus nach, innen zur Pfanne (20) verjüngt ausgebildet ist.

6. Gelenkprothese nach einem der Ansprüche 2 bis 5, dadurch gekennzeichnet, daß der Innendurchmesser (6) der Scheibe (2) kleiner als der des Pfannenrandes (14) ist.

## Claims

1. Artificial joint, more particularly an artificial hip joint, having a ball member (32) and a socket member (8) having an annular socket edge, the surface (34) of the ball member (32) abutting on an inner surface (20) of the sockel member (8), characterised in that, on the surface (16) of the socket edge, there is a disc (2) which is to be removed after the ball member (32) has been located in the socket member (8), or in that at least the surface (16) of the socket edge or the surface (34) of the ball member (32) is provided with a protective coating (28, 30) consisting of a material which can be absorbed by the body, and in that the material of the disc (2) or of the protective coating (28, 30) is less hard than the material of the socket member (8) or ball member (32).

2. Artificial joint according to claim 1, characterised in that the annularly formed disc (2) and/or the protective coating (28, 30) are of essentially constant thickness.

3. Artificial joint according to claim 2, characterised in that the disc (2) has at least one attachment (10) which interlockingly engages in a recess (12) in the socket member (8).

4. Artificial joint according to claim 2 or 3, characterised in that the disc (2) has a radial slot (4) passing through it.

5. Artificial joint according to one of claims 2 to 4, characterised in that the inner edge (14) of the disc (2) is configured to taper inwardly towards the socket (20), more particularly in the manner of a cone.

6. Artificial joint according to one of claims 2 to 5, characterised in that the internal diameter (6) of the disc (2) is smaller than that of the socket edge (14).

## Revendications

1. Prothèse d'articulation, notamment prothèse d'articulation de hanche, comprenant un corps sphérique (32) et un corps de cotyle (8) présentant un bord de cotyle annulaire, la surface (34) du corps sphérique (32) étant en appui contre une surface intérieure (20) du corps de cotyle (8), **caractérisée en ce que,** sur la surface (16) du bord du cotyle, est disposée une rondelle (2) destinée à être retirée après l'engagement du corps sphérique (32) dans le corps de cotyle (8) ou en ce qu'au moins la surface (16) du bord du cotyle ou la surface (34) du corps sphérique (32) est munie d'une couche protectrice (28, 30), qui est composée d'une matière pouvant être résorbée par le corps et, en ce que la matière de la rondelle (2), ou celle de la couche protectrice (28, 30), possède une plus faible dureté que la matière du corps (8) du cotyle ou que celle du corps sphérique (32) respectivement.

2. Prothèse d'articulation selon la Revendication 1, **caractérisée en ce que** la rondelle de forme annulaire (2) et/ou la couche protectrice (28, 30) présentent une épaisseur de couche sensiblement constante.

3. Prothèse d'articulation selon la Revendication 2, **caractérisée en ce que** la rondelle (2) présente au moins une patte (10) qui s'engage dans un évidement (12) du corps de cotyle (8) en établissant une liaison par sûreté de forme.

4. Prothèse d'articulation selon la Revendication 2 ou 3, **caractérisée en ce que** la rondelle (2) présente une fente radiale traversante (4).

5. Prothèse d'articulation selon l'une des Revendications 2 à 4, **caractérisée en ce que** le bord intérieur (14) de la rondelle (2) est d'une forme qui se rétrécie vers l'intérieur, en direction du cotyle (20), notamment avec une forme conique.

6. Prothèse d'articulation selon l'une des Revendications 2 à 5, **caractérisée en ce que** le diamètre intérieur (6) de la rondelle (2) est plus petit que celui du bord (14) du cotyle.

4

2

6

**Fig. 1**

20   28

8   26

12   24

10

16   22

14

**Fig. 2**

30

32

34

**Fig. 3**